# EUROPEAN PATENT APPLICATION

(11) **EP 2 742 870 A1**
(43) Date of publication of application: **18.06.2014**
(21) Application number: 12821704.9
(22) Date of filing: 03.08.2012
(51) Int. Cl.: A61B 8/12

(54) **ULTRASOUND PROBE**

(30) Priority: 08.08.2011 JP 2011172769
(71) Applicant: Konica Minolta, Inc., Tokyo 100-7015 (JP)
(72) Inventor: KUSAKA, Keigo, Osaka 540-6207 (JP)
(74) Representative: Gille Hrabal
(86) International application number: PCT/JP2012/004940
(87) International publication number: WO 2013/021598

(57) **Abstract**

An ultrasound probe according to the present disclosure includes: an ultrasound element unit 2; a tip storage portion 7 including a panel which is sealed by a holding member and in which an acoustic medium liquid 6 and the ultrasound element unit 2 are stored; a cover member 17a disposed on a surface of the holding member 10a; and an acoustic medium liquid reservoir portion 15a for retaining the acoustic medium liquid 6 in a space formed by the holding member 10a and the cover member 17a. The holding member 10a has at least one communication path that communicates with the acoustic medium liquid reservoir portion 15a and an inside of the tip storage portion 7. The cover member has at least one ventilation path that communicates with the acoustic medium liquid reservoir portion and an outside of the tip storage portion. In the acoustic medium liquid reservoir portion, a cross-sectional area of a space of a part that communicates with the ventilation path is larger than a cross-sectional area of a space of a part that communicates with the communication path.

## Description

### TECHNICAL FIELD

The present invention belongs to the technical field of ultrasound probes used in ultrasound diagnosis.

### BACKGROUND ART

An ultrasound probe for obtaining ultrasound tomograms is used in diagnosis of a tumor and the like in a body, and includes: a tip storage portion containing an ultrasound element for transmitting and receiving ultrasound waves, and the like; an insertion portion for inserting the tip storage portion into the body to a predetermined position; and a grip portion the whole of which is gripped for operation.

As such an ultrasound probe, an ultrasound probe in which the ultrasound element for transmitting and receiving ultrasound waves is rotated or swung inside the tip storage portion in which an acoustic medium liquid having an acoustic impedance similar to that of a living body is enclosed is known. In this ultrasound probe, the ultrasound element and a mechanism portion for rotating or swinging the ultrasound element are disposed in the tip storage portion.

A window made of a material through which ultrasound waves easily pass is provided in a part of the tip storage portion facing a wave transmission and reception surface of the ultrasound element. A gap between the wave transmission and reception surface of the ultrasound element and the window is filled with the acoustic medium liquid.

The acoustic medium liquid is used to acoustically match the wave transmission and reception surface of the ultrasound element and the window for efficient transmission and reception of ultrasound waves. In principle, only the gap between the wave transmission and reception surface of the ultrasound element and the window needs to be filled with the acoustic medium liquid. However, in practice it is difficult to fill only the gap with the acoustic medium liquid, and so a method of sealing a space containing the ultrasound element and filling the sealed space with the acoustic medium liquid is typically employed.

On the other hands, the acoustic medium liquid usually expands or contracts according to temperature. In the case where the sealed space is filled with the acoustic medium liquid, the expansion of the acoustic medium liquid causes an increase in internal pressure of a case forming the sealed space, which leads to troubles such as a leakage of the acoustic medium liquid, a crack, and so on. Besides, air bubbles may enter in the process of injecting the acoustic medium liquid into the sealed space.

If air bubbles are present between the ultrasound element and the window, there is a problem that transmitted and received ultrasound waves are attenuated or reflected by such air bubbles and as a result clear ultrasound tomograms are unable to be obtained.

To prevent such troubles, a diaphragm that is connected to the sealed space filled with the acoustic medium liquid and absorbs the expansion and contraction of the acoustic medium liquid is provided outside the sealed space (for example, Patent Document 1).

There is also a method of providing, in addition to the diaphragm, an air bubble reservoir portion for moving air bubbles out of the acoustic medium liquid-filled space by the differences in surface tension and specific gravity between the air bubbles and the acoustic medium liquid (for example, Patent Document 2).

As described above, an ultrasound probe having the following structure is used: a window is provided in a sealed space; an ultrasound element for transmitting and receiving ultrasound waves, a mechanism portion for rotating or swinging the ultrasound element, and the like are contained in the sealed space; the sealed space is filled with an acoustic medium liquid of an acoustic medium; and an air bubble reservoir portion and a diaphragm connected to the sealed space are provided outside the sealed space.

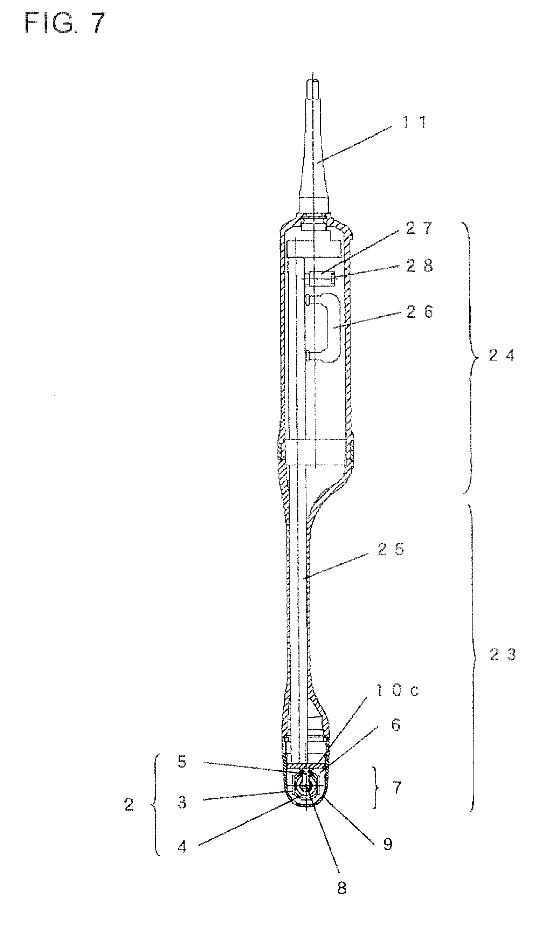

FIG. 7 is a sectional view showing an overall structure of a conventional ultrasound probe. The ultrasound probe includes: an insertion portion 23 including a tip storage portion 7 that is inserted into the body cavity; and a grip portion 24 gripped by an operator outside the body cavity.

The tip storage portion 7 is formed by joining a window 9 and a frame 10c, and stores an ultrasound element unit 2 inside.

The ultrasound element unit 2 includes: an ultrasound element 3; and a rotation mechanism portion for holding and rotating the ultrasound element 3. The rotation mechanism portion is a self-rotating motor whose rotation is magnetically induced, and includes: a motor rotor 4 carrying the ultrasound element 3; a bracket 5 for rotatably supporting the motor rotor 4 about a rotation center 8 of the motor rotor 4; and a magnet (not shown) for supplying a rotational force to the motor rotor 4. Such a rotation mechanism portion rotates the ultrasound element 3 with the rotation of the motor rotor 4, thus achieving mechanical ultrasound scanning in a circular orbit. Though not shown, a plurality of signal lines for transmitting and receiving electrical signals for driving the ultrasound element 3 and the rotation mechanism portion are drawn out of the ultrasound element unit 2, and led to the grip portion 24 through the insertion portion 23 and connected to a cable 11.

The bracket 5 of the rotation mechanism portion is fixed onto the frame 10c. The frame 10c is disposed so as to be in close contact with the inner wall of the window 9, and liquid-tightly seals the tip storage portion 7.

The tip storage portion 7 is filled with an acoustic medium liquid 6. A through hole is formed in the frame 10c, and a metal pipe 25 extending to the grip portion 24 through the insertion portion 23 is connected to the through hole. The metal pipe 25 is filled with the acoustic medium liquid 6, too.

A diaphragm 26 connected to the internal space of the metal pipe 25 and filled with the acoustic medium liquid 6 is attached to the metal pipe 25 filled with the acoustic medium liquid 6. The diaphragm 26 is made of an elastic material such as rubber, and absorbs the expansion and contraction of the acoustic medium liquid 6 enclosed in the metal pipe 25 and the tip storage portion 7 in order to prevent a liquid leakage or a member crack caused by the expansion of the acoustic medium liquid 6. The metal pipe 25 is provided with a liquid injection hole 27 for injecting the acoustic medium liquid 6, and the liquid injection hole 27 is closed by a liquid plug 28.

The cable 11 is drawn out of the grip portion 24, and the ultrasound probe is connected to an ultrasound diagnostic apparatus via the cable 11.

### PRIOR ART DOCUMENTS

### PATENT DOCUMENTS

Patent Document 1: Japanese Patent Application Publication No. 2004-298463
Patent Document 2: Japanese Patent Application Publication No. 2005-334107

### SUMMARY OF THE INVENTION

However, the conventional ultrasound probe has the following problem. When injecting the acoustic medium liquid into the tip storage portion, the acoustic medium liquid is injected into the liquid injection hole of the metal pipe connected to the tip storage portion and then the liquid injection hole is plugged. Upon plugging the liquid injection hole, air may enter the tip storage portion from the liquid injection hole. Besides, since the diaphragm is made of a material such as rubber, the rubber may deteriorate due to a temperature change and the like and be cracked, causing air to enter the tip storage portion through the diaphragm.

The air entering the tip storage portion forms air bubbles in the acoustic medium liquid. If air bubbles are present between the ultrasound element and the window in the tip storage portion, transmitted and received ultrasound waves are attenuated or reflected by such air bubbles, as a result of which high-quality ultrasound tomograms are unable to be obtained.

An aspect of the present disclosure solves the conventional problem, and provides a technique that enables high-quality ultrasound tomograms to be obtained by preventing air from entering the tip storage portion containing the ultrasound element.

An aspect of the present disclosure includes: an ultrasound element unit for transmitting and receiving an ultrasound wave; a tip storage portion including a panel which is sealed by a holding member and in which an acoustic medium liquid and the ultrasound element unit are stored; a cover member disposed on a surface of the holding member; and an acoustic medium liquid reservoir portion for retaining the acoustic medium liquid in a space formed by the holding member and the cover member, wherein the holding member has at least one communication path that communicates with the acoustic medium liquid reservoir portion and an inside of the tip storage portion, wherein the cover member has at least one ventilation path that communicates with the acoustic medium liquid reservoir portion and an outside of the tip storage portion, and wherein a cross-sectional area of the acoustic medium liquid reservoir portion is larger in a part that communicates with the ventilation path than in a part that communicates with the communication path.

According to an aspect of the present disclosure, the tip storage portion storing the ultrasound element unit is filled with the acoustic medium liquid, and a space for retaining the acoustic medium liquid from the communication path is formed in the acoustic medium liquid reservoir portion disposed adjacent to the tip storage portion via the communication path.

Thus, in the process of injecting the acoustic medium liquid into the tip storage portion of the ultrasound probe according to the present disclosure, the tip storage portion can be filled with the acoustic medium liquid without air bubbles remaining in the tip storage portion. As an injection method, the acoustic medium liquid of an amount specified from the space of the acoustic medium liquid reservoir portion is quantitatively injected into the tip storage portion from the ventilation path using a syringe or the like. Following this, by placing the structure under reduced pressure conditions, air bubbles in the tip storage portion which have entered during the injection of the acoustic medium liquid are expanded and exhausted out of the tip storage portion from the ventilation path for communication between the acoustic medium liquid reservoir portion and the outside air.

As a result, the acoustic medium liquid of an appropriate amount can be held in the acoustic medium liquid reservoir portion, and air bubbles can be prevented from remaining in the tip storage portion.

Moreover, the expansion and contraction of the acoustic medium liquid due to a temperature change during use of the ultrasound probe can be absorbed in the acoustic medium liquid reservoir portion.

In the ultrasound probe, the cross-sectional area of the space of the acoustic medium liquid reservoir portion changes from a minimum gap portion where the cross-sectional area of the space is smallest to a maximum gap portion where the cross-sectional area of the space is largest, in the circumferential direction. The communication path is formed near the minimum gap portion, whereas the ventilation path is formed near the maximum gap portion.

Here, a part of the acoustic medium liquid reservoir portion mentioned above communicates with the communication path. The acoustic medium liquid reservoir portion has, near the communication path in which the acoustic medium liquid is present, the minimum gap portion where the size (cross-sectional area) of the gap is smallest and, near the ventilation path, the maximum gap portion where the size of the gap is largest. The communication path and the ventilation path are formed near the minimum gap portion and the maximum gap portion, respectively.

Since the size of the gap in the acoustic medium liquid reservoir portion decreases from the ventilation path toward the communication path, a suction force in the direction opposite to the ventilation path can be generated by the surface tension of the acoustic medium liquid. As a result, the acoustic medium liquid can be retained in the acoustic medium liquid reservoir portion, while preventing the acoustic medium from leaking to the outside from the ventilation path.

In addition, according to the above-mentioned structure, the acoustic medium liquid reservoir portion has such a shape that inclines in the circumferential direction so that the height distance in the acoustic medium liquid reservoir portion increases from the minimum gap portion near the communication path toward the ventilation path. Therefore, even when an external shock is applied to the ultrasound probe or the position of the ultrasound probe abruptly changes, the interface between the air and the acoustic medium liquid in the acoustic medium liquid reservoir portion remains near the ventilation path, and is kept from moving in the circumferential direction. The leakage of the acoustic medium liquid to the outside with the movement of air bubbles can be suppressed in this way. Furthermore, the cross-sectional area of the retention space of the acoustic medium liquid reservoir portion is larger in a part nearer the ventilation path, and also the interface has a radially extending shape. Accordingly, the variation of the area of the interface and the variation of the surface tension associated with it are little.

Though the above describes a probe that uses an ultrasound element unit for mechanically scanning ultrasound waves as an example, the present disclosure is not limited to this, and may also be applied to an ultrasound probe that uses an ultrasound element unit for electronic scanning using an array element. The space shape in the acoustic medium liquid reservoir portion may be provided in a cover member or in a frame.

Such use of the space shape having the sealing function in the acoustic medium liquid reservoir portion eliminates the need of the pipe member, the diaphragm, the dedicated liquid injection hole, and the liquid plug of the conventional structure. This simplifies the mechanism portion in the ultrasound probe, and enables the components to be arranged in a smaller space and the number of components to be reduced. Hence, a more lightweight, smaller, and less expensive ultrasound probe can be realized as a whole.

Thus, according to an aspect of the present disclosure, it is possible to prevent air from entering the tip storage portion containing the ultrasound element, so that high-quality ultrasound tomograms can be obtained.

### BRIEF DESCRIPTION OF THE DRAWINGS

[FIG. 1] FIG. 1 is a sectional view showing an overall structure of an ultrasound probe in Embodiment 1 of the present disclosure.
[FIG. 2] FIG. 2 is a sectional view showing a structure in a tip storage portion of the ultrasound probe in Embodiment 1 of the present disclosure.
[FIG. 3] FIG. 3 is an external perspective view of an ultrasound diagnostic apparatus using the ultrasound probe in Embodiment 1 of the present disclosure.
[FIG. 4A] FIG. 4A is a perspective view showing a gap space in the tip storage portion of the ultrasound probe in Embodiment 1 of the present disclosure.
[FIG. 4B] FIG. 4B is a sectional view showing the gap space in the tip storage portion of the ultrasound probe in Embodiment 1 of the present disclosure.
[FIG. 5] FIG. 5 is a sectional view showing a structure in a tip storage portion of an ultrasound probe in Embodiment 2 of the present disclosure.
[FIG. 6] FIG. 6 is a perspective view conceptually showing a gap space in the tip storage portion of the ultrasound probe in Embodiment 2 of the present disclosure.
[FIG. 7] FIG. 7 is a sectional view showing an overall structure of a conventional ultrasound probe.

### MODES FOR CARRYING OUT THE INVENTION

The following describes embodiments specifically showing best modes for carrying out the present disclosure, with reference to drawings.

### (Embodiment 1)

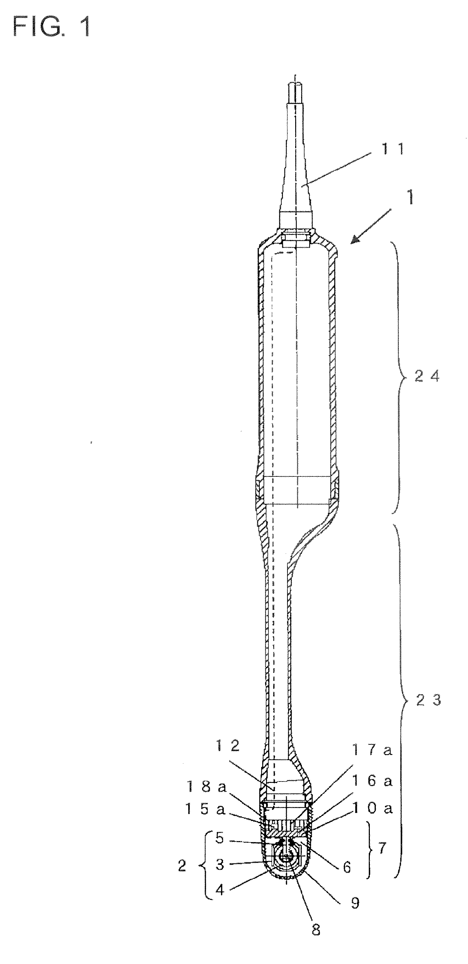

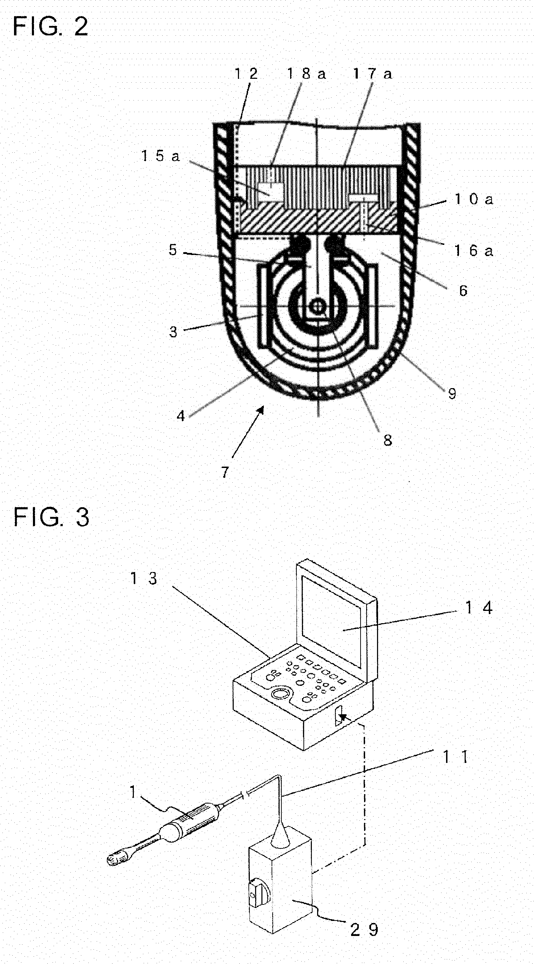

FIG. 1 is a sectional view showing an example of a structure of an ultrasound probe 1 according to the present disclosure. FIG. 2 is an enlarged sectional view of a tip storage portion 7 in FIG. 1. FIG. 3 is an external perspective view of an ultrasound diagnostic apparatus using the ultrasound probe 1. Note that the same components as those in the conventional ultrasound probe shown in FIG. 7 are given the same reference numerals.

In FIG. 1, the ultrasound probe 1 is a probe used in ultrasound diagnosis, and is a body cavity insertion-type probe which is partly inserted into a subject's body cavity to perform ultrasound scanning in the body cavity. The ultrasound probe includes: an insertion portion 23 including a tip storage portion 7 that is inserted into the body cavity; and a grip portion 24 gripped by an operator outside the body cavity. The direction from the grip portion 24 to the insertion portion 23 of the ultrasound probe 1 is hereafter referred to as "insertion axial direction".

The tip storage portion 7 is formed by joining a panel (hereafter referred to as "window") 9 and a holding member (hereafter referred to as "frame") 10a, and has: a space portion in which an ultrasound element unit 2 is stored; and a space portion serving as an acoustic medium liquid reservoir portion 15a formed by joining a cover member 17a and the frame 10a.

The ultrasound element unit 2 includes: an ultrasound element 3; and a rotation mechanism portion for holding and rotating the ultrasound element 3, as shown in FIG. 2. The rotation mechanism portion is a self-rotating motor whose rotation is magnetically induced, and includes: a motor rotor 4 carrying the ultrasound element 3; a bracket 5 fixed to the frame 10a for rotatably supporting the motor rotor 4 about a rotation center 8 of the motor rotor 4; and a magnet (not shown) for supplying a rotational force to the motor rotor 4. Such a rotation mechanism portion rotates the ultrasound element 3 with the rotation of the motor rotor 4, thus achieving mechanical ultrasound scanning in a circular orbit.

A plurality of signal lines 12 for transmitting and receiving electrical signals for driving the ultrasound element 3 and the rotation mechanism portion are drawn out of the ultrasound element unit 2. The signal lines 12 pass through a wiring hole provided in the frame 10a, and are led to the grip portion 24 through the insertion portion 23 and connected to a cable 11.

The cable 11 is drawn out of the grip portion 24, and the ultrasound probe 1 is connected to an ultrasound diagnostic apparatus 13 in FIG. 3 via the cable 11.

In FIG. 3, an electrical signal (transmission signal) from the ultrasound diagnostic apparatus 13 is transmitted to the ultrasound element 3 of the ultrasound probe 1 through the cable 11 that is attached to a connecter 29 connected to the ultrasound diagnostic apparatus 13. The transmission signal is converted to an ultrasound wave in the ultrasound element 3 in FIG. 2, propagates through an acoustic medium liquid 6, and is transmitted from the window 9 to the subject. The ultrasound wave is reflected in the subject. A part of the reflected wave is received by the ultrasound element 3 and converted to an electrical signal (reception signal), and transmitted to the ultrasound diagnostic apparatus 13 in FIG. 3. The reception signal is converted to image data and displayed on a display 14 in the ultrasound diagnostic apparatus 13.

Since the tip storage portion 7 is liquid-tightly sealed, the signal lines 12 are sealed by an adhesive or the like in the wiring hole of the frame 10a disposed so as to be in close contact with the inner wall of the window 9. The tip storage portion 7 is filled with the acoustic medium liquid 6.

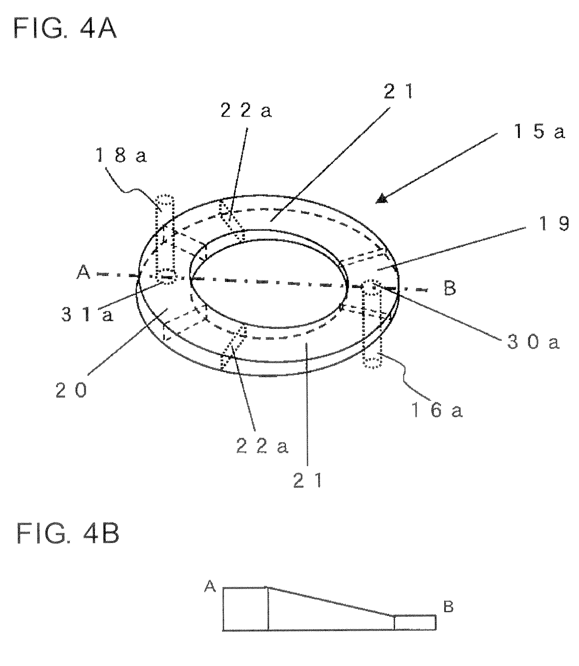

The tip storage portion 7 of the ultrasound probe 1 in Embodiment 1 of the present disclosure is described in detail below, with reference to FIGS. 2, 4A, and 4B.

FIG. 4A is a perspective view showing a shape of the space (the acoustic medium liquid reservoir portion 15a) enclosed by the frame 10a and the cover member 17a in FIG. 2. FIG. 4B is a sectional view of the space when the acoustic medium liquid reservoir portion 15a in FIG. 4A is cut along line AB.

The space shape of the acoustic medium liquid reservoir portion 15a is such a shape that inclines in the circumferential direction so that the separation distance between the cover member 17a and the frame 10a in the acoustic medium liquid reservoir portion 15a increases from a minimum gap space portion 19 toward a maximum gap space portion 20 connected to a ventilation path 18a. Accordingly, the cross-sectional area of the space of the part that communicates with the ventilation path 18a is larger than the cross-sectional area of the space of the part that communicates with a communication path 16a.

In the space shape of the acoustic medium liquid reservoir portion 15a, there are the minimum gap space portion 19 connected to the communication path 16a provided in the frame 10a in FIG. 2 and the maximum gap space portion 20 connected to the ventilation path 18a provided in the cover member 17a, as shown in the perspective view in FIG. 4A. Moreover, an inclined space portion 21 is included in the space sandwiched between the minimum gap space portion 19 and the maximum gap space portion 20 of the acoustic medium liquid reservoir portion 15a. The inclined space portion 21 is shaped to incline in the circumferential direction perpendicular to the insertion axial direction so that the separation distance between the cover member 17a and the frame 10a increases from the minimum gap space portion 19 toward the maximum gap space portion 20. The communication path 16a and the ventilation path 18a in FIG. 2 are connected to the acoustic medium liquid reservoir portion 15a at a communication path position 30a and a ventilation path position 31 a in FIG. 4A, respectively.

Note that the thickness in the insertion axial direction of the minimum gap space portion 19 in the acoustic medium liquid reservoir portion 15a on the communication path 16a side is smaller than the thickness in the insertion axial direction of the maximum gap space portion 20, and smallest so as to produce a capillary phenomenon. On the other hand, the maximum gap space portion 20 in the acoustic medium liquid reservoir portion 15a on the ventilation path 18a side has such a size that does not produce any capillary phenomenon, as the gap gradually increases in the circumferential direction perpendicular to the insertion axial direction from the minimum gap space portion 19 toward the maximum gap space portion 20.

The following describes a procedure of filling the tip storage portion 7 with the acoustic medium liquid 6. The filling amount of the acoustic medium liquid 6 is set so that the acoustic medium liquid 6 does not enter the maximum gap space portion 20 even in a state where the acoustic medium liquid 6 expands at the maximum in a use environment. That is, a gas-liquid interface 22a in FIG. 4A, which is the boundary between the air and the acoustic medium liquid 6, is positioned within the inclined space portion 21. This is because the acoustic medium liquid 6 and the air are separated from each other in the acoustic medium liquid reservoir portion 15a due to their differences in surface tension and specific gravity. Thus, the acoustic medium liquid 6 gathers at the narrow gap near the communication path 16a due to the capillary phenomenon, while the air gathers at the wide space in the acoustic medium liquid reservoir portion 15a.

Therefore, even in the case of a temperature rise in the use environment of the ultrasound probe 1 or the like, the gas-liquid interface 22a of the acoustic medium liquid 6 does not enter the maximum gap space portion 20, so that the acoustic medium liquid 6 can be prevented from leaking from the ventilation path 18a in FIG. 2.

As described above, according to the present disclosure, even when an external shock is applied to the ultrasound probe 1 or the position of the ultrasound probe 1 abruptly changes, the gas-liquid interface 22a between the air and the acoustic medium liquid 6 in the acoustic medium liquid reservoir portion 15a remains in the inclined space portion 21 due to the capillary phenomenon, so that the air can be prevented from entering the minimum gap space portion 19.

Since the air does not enter the space enclosed by the window 9 and the frame 10a and filled with the acoustic medium liquid 6 through the ventilation path 18a, the generation of air bubbles from such air in the tip storage portion 7 containing the ultrasound element 3 can be suppressed.

Though one communication path 16a and one ventilation path 18a are provided in this example, a plurality of communication paths and a plurality of ventilation paths may be provided.

In this example, the ventilation path 18a is set at a position apart from the communication path 16a in approximate point symmetry with respect to the center of the acoustic medium liquid reservoir portion 15a.

This, in combination with the surface tension sealing function by the taper shape of the acoustic medium liquid reservoir portion 15a, allows the acoustic medium liquid 6 to be retained in the acoustic medium liquid reservoir portion 15a.

Moreover, by making the cover member 17a of a translucent material, the acoustic medium liquid 6 can be visually observed through the cover member 17a when filling the tip storage portion 7 with the acoustic medium liquid 6. In detail, in a plan view where the tip storage portion 7 in FIG. 2 is viewed in the downward direction of the paper surface, i.e. in the insertion axial direction, the acoustic medium liquid 6 is retained in a substantial C-shape in a plane approximately perpendicular to the insertion axial direction in the acoustic medium liquid reservoir portion 15a. Here, the open end of the C-shape of the acoustic medium liquid reservoir portion 15a is the gas-liquid interface 22a in the acoustic medium liquid reservoir portion 15a.

This being so, when expanding the acoustic medium liquid 6 at the maximum in the use environment and filling the tip storage portion 7 with the acoustic medium liquid 6, the gas-liquid interface 22a can be kept from entering the maximum gap space portion 20 by visually observing the above-mentioned open part of the C-shape through the cover member 17a made of a transparent material. As a result, the tip storage portion 7 can be filled with the acoustic medium liquid 6, and also the acoustic medium liquid 6 of an appropriate amount can be retained in the acoustic medium liquid reservoir portion 15a.

As described above, in Embodiment 1, the acoustic medium liquid 6 is retained in the acoustic medium liquid reservoir portion 15a, so that the air can be prevented from entering the tip storage portion 7. An ultrasound probe capable of obtaining high-quality ultrasound tomograms can thus be provided.

### (Embodiment 2)

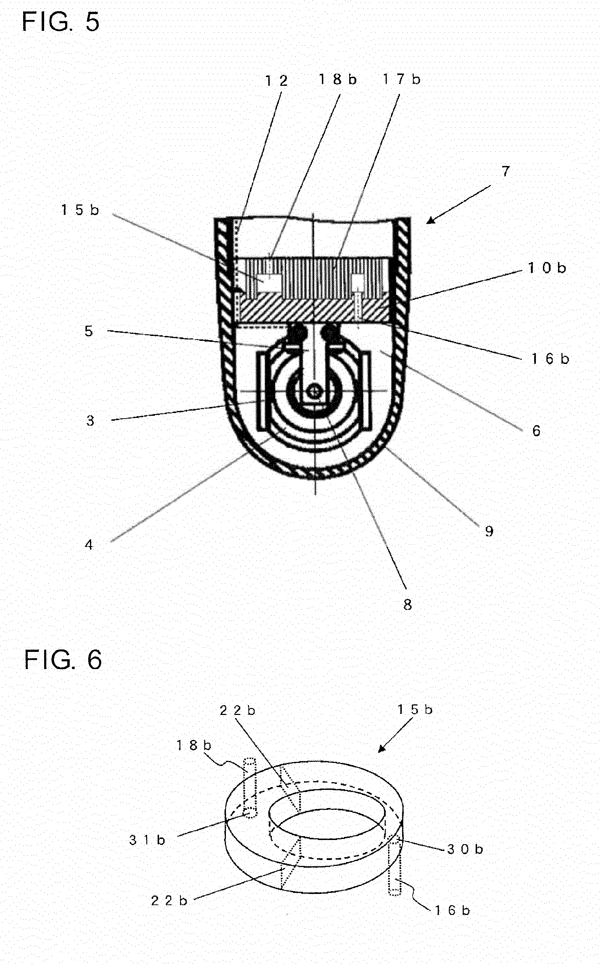

The following describes a second embodiment of the present invention with reference to FIGS. 5 and 6.

FIG. 5 is a sectional view showing a structure in the tip storage portion 7 of the ultrasound probe according to the present disclosure. FIG. 6 is a perspective view conceptually showing a space shape in an acoustic medium liquid reservoir portion 15b and a positional relationship between a ventilation path 18b and a communication path 16b in Embodiment 2.

The space shape of the acoustic medium liquid reservoir portion 15b in FIG. 5 is such that the radial gap gradually changes in the circumferential direction, as shown in the perspective view in FIG. 6. That is, when comparing the planar shape of the acoustic medium liquid reservoir portion 15b to a double circle, the center of the inner circle is closer to a communication path position 30b than a ventilation path position 31 b. Accordingly, the distance between the inner circle and the outer circle of the double circle decreases from the ventilation path position 31b toward the communication path position 30b, so as to produce a capillary phenomenon in the direction perpendicular to the insertion axial direction. Since the acoustic medium liquid 6 in the acoustic medium liquid reservoir portion 15b is subject to the sealing function by the surface tension from the ventilation path 18b side to the communication path 16b side, the acoustic medium liquid 6 is retained in the acoustic medium liquid reservoir portion 15a, as in Embodiment 1.

The filling amount of the acoustic medium liquid 6 is set to position a gas-liquid interface 22b of the acoustic medium liquid 6 in consideration of the volume expansion coefficient of the acoustic medium liquid 6 and the thermal expansion coefficient of the component of the tip storage portion 7 so that, even in the case of a temperature rise in the installation environment of the ultrasound probe in a state of filling with the acoustic medium liquid 6, the gas-liquid interface 22b of the acoustic medium liquid 6 does not reach the ventilation path 18b and leak from the ventilation path 18b.

In Embodiment 2, the acoustic medium liquid 6 can be stably held in the acoustic medium liquid reservoir portion 15b, with the gas-liquid interface 22b as the boundary. In addition, air bubbles reliably undergo gas-liquid separation and move toward the larger gap connected to the ventilation path 18b in the acoustic medium liquid reservoir portion 15b. As a result, even in the structure in which the acoustic medium liquid reservoir portion 15b communicates with the outside air via the ventilation path 18b, the acoustic medium liquid 6 in the acoustic medium liquid reservoir portion 15b is always retained in the smaller gap, and kept from leaking to the outside from the ventilation path 18b. Besides, the air portion in the acoustic medium liquid reservoir portion 15b does not move regardless of the position of the ultrasound probe.

In the acoustic medium liquid reservoir portion 15b, the acoustic medium liquid 6 and the air separate from each other due to their differences in surface tension and specific gravity. The acoustic medium liquid 6 gathers at the narrow gap near the communication path 16b in the frame 10b due to the capillary phenomenon, while the air gathers at the wide space in the acoustic medium liquid reservoir portion 15b.

In particular, according to the present disclosure, even when an external shock is applied to the ultrasound probe or the position of the ultrasound probe abruptly changes, the acoustic medium liquid 6 can be prevented from moving in the circumferential direction to the ventilation path 18b side having the larger gap by the effect of the surface tension on the communication path 16b side having the smaller gap, as in Embodiment 1. Therefore, even when an external shock is applied to the ultrasound probe in the case where the acoustic medium liquid reservoir portion 15b in which the acoustic medium liquid 6 is retained communicates with the outside air via the ventilation path 18b, the leakage of the acoustic medium liquid 6 from the ventilation path 18b can be effectively suppressed.

Though one communication path 16b and one ventilation path 18b are provided in this example, a plurality of communication paths and a plurality of ventilation paths may be provided. In this example, the ventilation path 18b is set at a position apart from the communication path 16b in approximate point symmetry with respect to the center of the acoustic medium liquid reservoir portion 15b. Such a position is most preferable in terms of suppressing the leakage of the acoustic medium liquid 6 from the ventilation path 18b. This, in combination with the sealing function by the shape of the acoustic medium liquid reservoir portion 15b, enables more effective prevention of the acoustic medium liquid 6 retained in the acoustic medium liquid reservoir portion 15b from leaking from the ventilation path 18b.

Moreover, by making a cover member 17b of a translucent material, the acoustic medium liquid 6 can be visually observed through the cover member 17b when filling the tip storage portion 7 with the acoustic medium liquid 6.

In detail, in a plane view, the acoustic medium liquid 6 is retained in a substantial C-shape in the acoustic medium liquid reservoir portion 15b.

This being so, in the process of filling the tip storage portion 7 with the acoustic medium liquid 6, the acoustic medium liquid 6 of an appropriate amount can be retained by observing a liquid-level change in the acoustic medium liquid reservoir portion 15b.

As described above, in Embodiment 2, the acoustic medium liquid 6 is retained in the acoustic medium liquid reservoir portion 15b, so that the air can be prevented from entering the tip storage portion 7. An ultrasound probe capable of obtaining high-quality ultrasound tomograms can thus be provided.

According to an aspect of the present disclosure, a gas region may be present in the acoustic medium liquid reservoir portion, wherein the ventilation path is connected to the gas region.

Moreover, according to an aspect of the present disclosure, the cross-sectional area of the acoustic medium liquid reservoir portion may continuously decrease from the part that communicates with the ventilation path toward the part that communicates with the communication path.

Moreover, according to an aspect of the present disclosure, the acoustic medium liquid reservoir portion may be formed so that a radial gap changes in a circumferential direction of the acoustic medium liquid reservoir portion.

Moreover, according to an aspect of the present disclosure, the gap may be larger in the part that communicates with the ventilation path than in the part that communicates with the communication path.

Moreover, according to an aspect of the present disclosure, the ventilation path may be set at a position apart from the communication path with respect to, as a point of symmetry, a center point of the acoustic medium liquid reservoir portion. Thus, the capacity of the acoustic medium liquid reservoir portion for suppressing the leakage of the acoustic medium liquid from the ventilation path caused by the volume expansion of the acoustic medium liquid due to a temperature change can be secured easily.

Moreover, according to an aspect of the present disclosure, the cover member may be made of a translucent material. This enables the injection amount to be adjusted so that the gas-liquid interface of the acoustic medium liquid is situated at a predetermined position in the space in the acoustic medium liquid reservoir portion, while observing the position of the gas-liquid interface of the acoustic medium liquid in the acoustic medium liquid reservoir portion from the outside. Therefore, the filling amount of the acoustic medium liquid in the acoustic medium liquid reservoir portion can be managed accurately.

It is also possible to ensure the complete filling with the acoustic medium liquid by observing, after the injection of the acoustic medium liquid into the acoustic medium liquid reservoir portion, that the position of the gas-liquid interface in the acoustic medium liquid reservoir portion is unchanged under reduced pressure conditions. Note that the translucent member for the acoustic medium liquid reservoir portion may be used for the whole or part of the acoustic medium liquid reservoir portion.

Moreover, according to an aspect of the present disclosure, a liquid region may be present in the acoustic medium liquid reservoir portion, wherein the communication path is connected to the liquid region.

### INDUSTRIAL APPLICABILITY

The present disclosure is applicable to an ultrasound probe that does not allow air bubbles remaining in an acoustic medium liquid.

## Claims

1. An ultrasound probe comprising:
an ultrasound element unit for transmitting and receiving an ultrasound wave;
a tip storage portion in which an acoustic medium liquid and the ultrasound element unit are stored in a panel and which is sealed by a holding member;
a cover member disposed on a surface of the holding member; and
an acoustic medium liquid reservoir portion for retaining the acoustic medium liquid in a space formed by the holding member and the cover member,
wherein the holding member has at least one communication path that communicates with the acoustic medium liquid reservoir portion and an inside of the tip storage portion,
wherein the cover member has at least one ventilation path that communicates with the acoustic medium liquid reservoir portion and an outside of the tip storage portion, and
wherein a cross-sectional area of the acoustic medium liquid reservoir portion is larger in a part that communicates with the ventilation path than in a part that communicates with the communication path.

2. The ultrasound probe according to claim 1, wherein a gas region is present in the acoustic medium liquid reservoir portion, and the ventilation path is connected to the gas region.

3. The ultrasound probe according to claim 1 or 2, wherein the cross-sectional area of the acoustic medium liquid reservoir portion continuously decreases from the part that communicates with the ventilation path toward the part that communicates with the communication path.

4. The ultrasound probe according to any one of claims 1 to 3, wherein the acoustic medium liquid reservoir portion is formed so that a radial gap changes in a circumferential direction of the acoustic medium liquid reservoir portion.

5. The ultrasound probe according to claim 4, wherein the gap is larger in the part that communicates with the ventilation path than in the part that communicates with the communication path.

6. The ultrasound probe according to any one of claims 1 to 5, wherein the ventilation path is set at a position apart from the communication path with respect to, as a point of symmetry, a center point of the acoustic medium liquid reservoir portion.

7. The ultrasound probe according to any one of claims 1 to 6, wherein the cover member is made of a translucent material.

8. The ultrasound probe according to any one of claims 1 to 7, wherein a liquid region is present in the acoustic medium liquid reservoir portion, and the communication path is connected to the liquid region.
